Europäisches Patentamt

European Patent Office

Office européen des brevets

(19)

(11) Publication number: **0 064 828**
**B1**

(12) # EUROPEAN PATENT SPECIFICATION

(45) Date of publication of patent specification: **17.04.85**

(51) Int. Cl.⁴: **G 01 N 1/00, C 12 M 1/26**

(21) Application number: **82302098.7**

(22) Date of filing: **23.04.82**

(54) **Radial flow cell.**

(30) Priority: **27.04.81 US 258109**

(43) Date of publication of application:
**17.11.82 Bulletin 82/46**

(45) Publication of the grant of the patent:
**17.04.85 Bulletin 85/16**

(84) Designated Contracting States:
**BE DE FR GB IT NL**

(56) References cited:
**DE-A-2 844 841**
**DE-B-1 598 080**
**FR-A-2 121 827**
**US-A-3 754 868**

(73) Proprietor: **MERCK & CO. INC.**
**126, East Lincoln Avenue P.O. Box 2000**
**Rahway New Jersey 07065 (US)**

(72) Inventor: **Dobna, Robert W.**
**212 Central Avenue**
**Rahway New Jersey 07065 (US)**
Inventor: **Mayles, Allen B.**
**9 Sherman Court**
**Manalapan New Jersey 07726 (US)**
Inventor: **Stoudt, Thomas H.**
**857 Village Green**
**Westfield New Jersey 07090 (US)**

(74) Representative: **Crampton, Keith John Allen**
**et al**
**D YOUNG & CO 10 Staple Inn**
**London WC1V 7RD (GB)**

Courier Press, Leamington Spa, England.

## Description

Conventional methods of sampling industrial reaction vessels, such as fermentors, involve the aseptic and manual removal of individual portions through a sample port connected to the vessel into a receiver, most commonly a test tube or a flask. As it is essential to obtain representative samples, heterogeneous material must be taken directly from the stirring reaction mixture for sampling. It is difficult to filter the heterogeneous material, either due to high viscosity or complexity of constituents of different sizes and textures, and it can usually be removed efficiently only by manual methods. A material that it is notoriously difficult to filter is a fermentation broth with an unusually high percentage of solids, which are routinely removed aseptically and manually into a receiver protected from contamination only by a cotton plug. The individual samples are subsequently measured, filtered or centrifuged and stored until the required analysis can be performed.

The conventional method is inherently unsatisfactory because replacement of receivers for obtaining consecutive samples for analysis involves a high risk of contamination and the necessary delays incurred during the course of the conventional method, i.e., the time-consuming manual removal, separate filtration, storage prior to analysis, and subsequent manual dilution of samples, are sources of inaccuracy. The delayed analysis may not correspond to the course of the fermentation in real time, and the excessively manipulated samples may not give the true analysis of the fermentation broth due to contamination or decomposition. Furthermore, total on-line automation and control of the process is impossible with manual sampling.

Automation of an industrial fermentation process makes it a highly economic production process by avoiding overfermentation. It saves time and energy. It also eliminates the risk of spoiling the fermentation in cases where sensitive products are involved that may decompose if the fermentation is not terminated in time.

Most semi-automated biochemical processes, for example, fermentation, involve an off-line autoanalyser which analyses samples while monitoring the progress of the reaction. However, samples continuously taken from the reaction mixture for sampling must be properly filtered or centrifuged and diluted before entering the autoanalyser in order to bring the concentrations of analysed components within the range of the STDS and to avoid clogging of the instrument.

It is well known how difficult it can be to conduct a continuous and efficient filtration of a viscous heterogeneous reaction mixture, especially when it involves media having a high content of solids and when the fermentation lasts for days. Thus, even though some commercially available high-efficiency radial flow-cells have been used for filtering solutions containing extracts or casein from dairy products, no attempts at direct aseptic continuous filtration of a whole fermentation broth have been fully successful.

The present invention provides a radial flow cell of the type having a feed plate with inlet and outlet means for a heterogeneous liquid requiring filtration; a circular groove for receiving via connecting orifices the liquid from the inlet means and thereby generating a radial flow of broth; a filtrate plate with means for filtration; and a sealing means for securing the feed plate and the filtrate plate together so as to form a leak-proof cell, characterized in that the feed plate has two or more inlet ports for feeding the liquid; a dam attached to the periphery of the bottom surface of the feed plate and situated near connecting orifices connected to the inlet ports for enlarging the radius of the radial flow from the circular groove; and two or more outlet ports with enlarged openings in the feed plate to facilitate the exit of liquid containing a higher concentration of filterable-off material than the starting liquid.

A cell in accordance with the invention is capable of continuous and aseptic filtration, for a relatively long time, of a reaction mixture of similar viscosity and complexity of texture as a typical fermentation broth. Thus, the cell can be used in automatically monitoring the progress of a reaction by filtering a continuous flow of the reaction mixture during the entire reaction period; periodically removing discrete samples from the continuously flowing filtrate stream and feeding them to analysers in order to monitor the progress of the reaction; returning to the reaction vessel the residue swept from the surface of a biological membrane by a recirculating radial flow of the process stream; and returning the unused sterile filtrate to the reaction vessel. For example, the present invention could be used to incorporate the automatic monitoring method into a computer-controlled system for the automation of the reaction of the fermentation process.

The invention will be better understood by considering the preferred but illustrative embodiment shown in the first six figures of the accompanying drawings, in which:—

Fig. 1 is a perspective view of an assembled radial flow filter;

Fig. 2 is an exploded perspective view of a radial flow filter showing each and every part to be assembled;

Fig. 3 is a bottom plane view of a feed plate in a radial flow filter;

Fig. 4 is a cross-sectional view of the feed plate taken along the line 4—4 of Fig. 3;

Fig. 5 is a cross-sectional view of the feed plate taken along the line 5—5 of Fig. 3; and

Fig. 6 is an enlarged sectional view showing detail of an assembled radial flow filter.

Fig. 7 is an exploded perspective view of a known radial flow filter, the "Nucleopore Cell", which forms no part of the present invention. According to Fig. 7, the Nucleopore Cell consists of a feed plate 3, a filtrate plate 20 and a sealing means, e.g., a pressure clamp (not shown). The feed plate 3 has an inlet port 44 plus an outlet port 45 for recirculation. In order to generate a radial flow, a first port, be it inlet or outlet, penetrates through the body of the plate until it reaches an opening 43 extending downwardly through the centre of the plate, while the second port leads to an orifice 46 at the bottom of a concentric circular groove 6 having a diameter of about two-thirds of the diameter of the plate. The feed plate has, as an integral part, a downwardly extending cylindrical portion (column 8) adapted for engaging sealably with the inner wall of the filtrate plate and sealed to it, in operation, by means of an O-ring 52. Within the column 8 are located the previously described concentric circular groove 6 and the centre-located extended portion 43 of the first port. Both the groove and the first port have their openings facing downwardly at the bottom of the column.

The filtrate plate 20 has a shallow cylindrical cavity 90 for receiving sealably the column of the feeding plate. It holds support disc 50 (which is made of porous polyethylene), membrane 51 and the O-ring 52. It also has a filtrate output port 53 at the centre of its bottom. The radial flow cell is assembled by inserting the column of the top feeding plate into the cavity of the lower filtrate plate and sealing them together by means of the O-ring. The sealing is secured by pressing the plates tightly together with an adjustable clamp.

When in use, the process stream is continuously introduced into the assembled flow cell from the inlet port 44 and is to be recirculated in a radial flow across the surface of the membrane 51, thereby sweeping the membrane clean of any residual particles which may block the membrane pores. The particles are concentrated in the process stream while the filtrate passes through the continuously cleansed membrane due to a drop in pressure across the membrane, which is partially aided by a pump connected to the filtrate line.

However in reality, when the Nucleopore Cell is used for in-line filtration of heterogeneous materials of texture similar to that of a fermentation broth, only a partial radial flow covering about one-third of the membrane surface is generated, leaving the rest of the surface subject to gradual blocking. Eventually the residue reaches such a level as to hinder the radial flow over the entire membrane surface causing total stoppage of the filtration.

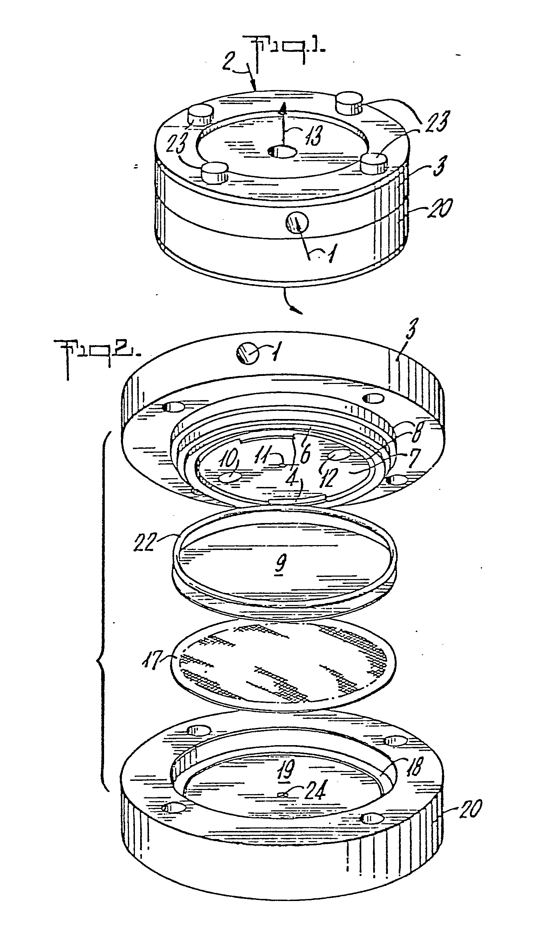

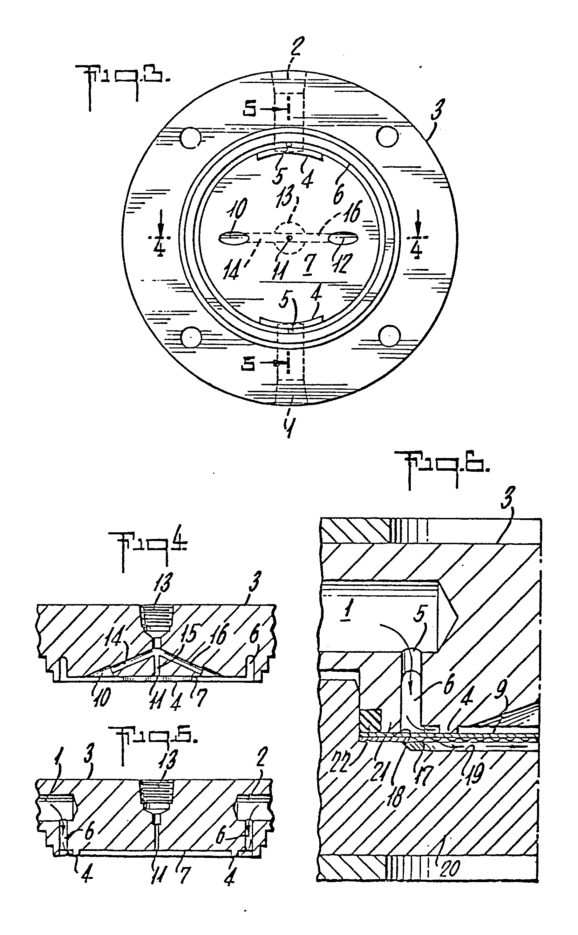

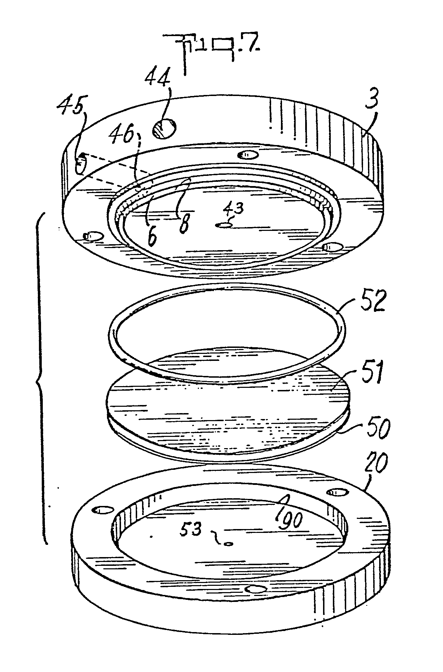

The radial flow cell of the present invention operates in substantially the same manner as the Nucleopore Cell described above but resolves the blocking problem by increasing the efficiency of the radial-flow cleansing function and of the filtration. The former is increased by adding one or more strategically positioned inlet ports, (ports 1 and 2 of Fig. 1), to the feed plate 3 so as to generate overlapping radial flows which would cover the entire micropore membrane surface 9 (Fig. 2); and by enlarging and increasing the number of the openings (10, 11, and 12 in Figs. 3 and 4) of outlet port 13 at the bottom surface of column 8 to facilitate the exit of the process stream which has its concentration of residual material increased by that swept off the membrane surface 9. This is accomplished by branching the outlet port into three or more branch ports (14, 15, 16 in Fig. 4): each extends downwardly through column 8 and has openings 10, 11, and 12 at the bottom surface of the column. Two of these openings 10 and 12 are enlarged by means of boring through the surface at an angle of about 15—30°.

The efficiency of the filtration is improved by installing a dam (4 in Figs. 2 and 3) near the orifice 5 at the bottom of the circular groove 6 for the purpose of spreading the incoming process flow to a larger radial area. In order to accomplish this effect, the dams are situated at the periphery of the lower bottom portion 7 of column 8 which constitutes the bottom surface of the feed plate 3. When the process stream is introduced via the inlet port 1 (Fig. 6), it flows first through the orifice 5 into the circular groove 6 (Fig. 6). Before leaving the groove, a major portion of the stream hits the dam 4 and is forced to spread around the dam. This automatically enlarges the radius of the flow which then sweeps through more than half of the membrane surface 9. Accordingly, a combination of two or more sets of the inlet port-groove-dam device such as shown in Fig. 6, will generate an efficient radial flow capable of sweeping and cleansing the entire surface of the membrane. As a result, the micropore membrane can be used continuously for the entire in-line filtration period without troublesome blockage.

The real-time sampling of filtrate has been improved by reducing the filtrate reservoir 19 (Fig. 2) area to ensure complete exchange of sample between sampling periods and eliminate timed-sample interaction and by replacing the supporting disc in the Nucleopore Cell with a stainless steel screen (17 in Fig. 2) of much larger pores but much smaller retention volume. The screen is supported by a circular shoulder 18 extending upwardly from the bottom surface 19 (filtrate reservoir) of the cavity of the filtrate plate 20. The screen 17 serves to support the micropore membrane without over-retention of filtrate.

Besides the three major improvements described above, the present invention also makes possible a modification of the sealing means for joining together the feeding and filter plates. The thickness of the lower outer wall (21 in Fig. 6) flanging the circular groove 6 is increased from about 0.05 cm to about 0.15 cm. The strengthened wall can withstand the sealing pressure exerted by O-ring 22 (Fig. 2), without breakage, for a much longer period of time than the thin (0.05 cm) wall of the Nucleopore Cell. It thus serves the purpose of preventing leakage during continuous in-line filtration, which may last for many days.

Finally, to eliminate the bulky and cumbersome clamp, the plates 3 and 20 are secured together by means of nuts 23 (Fig. 1) screwed onto bolts, which also provides a means for compressing the O-ring seal to make it leak-proof.

The following examples illustrate the efficiency of the improved radial flow cell as it is claimed.

### Example 1

The fermentation broth resulting from a 10-liter fermentation of *S. lactamdurans* in a Cerelose medium (see Table I for composition of the medium) for 14 days is filtered through the radial flow cell of the present invention. The following result is obtained:

Effective whole broth flow rates used: 150—1400 ml/min.

Filtrate flow rate achieved: 0.1—0.42 ml/min.

Duration of operation: 14 days. At the time when the filtration is stopped, there is no clogging of the membrane.

### Table I

#### Composition of Cerelose Medium

|  | Gram per Liter |
|---|---|
| Meat Meal (30 mesh) | 15 |
| Cerelose | 60 |
| DMF | 10 |
| $MgSO_4 \cdot 7H_2O$ | 0.5 |
| Glycine | 1.0 |
| P-2000 | 0.25 |
| pH with 25% NaOH | 7.3 |
| Sodium thiosulphate | 1.6 |
| D,L-Lysine · HCl | 0.98 |
| 1,3-diaminopropane ('Cerelose' is a trade mark) | 0.50 |

### Example 2

The fermentation broth resulting from a 10-liter fermentation of *S. cattleya* in a glycerol medium (see Table II for composition of the medium) for 14 days is filtered through the radial flow cell of the present invention.

The following result is obtained:

Effective whole broth flow rates used: 150—1400 ml/min.

Filtrate flow rate achieved: 0.1—0.42 ml/min.

Duration of operation: 14 days. At the time when the filtration is stopped, there is no clogging of the membrane.

### Table II

#### Composition of Sucrose Medium

|  | Gram per Liter |
|---|---|
| Glycerol | 20 |
| Distillers' solubles | 10 |
| Corn-steep liquor | 15 |
| Proflo | 5 |
| $CoCl_2 \cdot 6H_2O$ | 0.01 |
| Sodium succinate | 1 |
| pH with 50% caustic soda | 7.5 |
| $CaHPO_4$ | 2.5 |
| P-2000 | 0.25 |

### Claims

1. A radial flow cell of the type having a feed plate (3) with inlet and outlet means for a heterogeneous liquid requiring filtration; a circular groove (6) for receiving via connecting orifices the said liquid from the inlet means and thereby generating a radial flow of the said liquid; a filtrate plate (20) with means for filtration (9); and a sealing means (22) for securing the feed plate (3) and the filtrate plate (20) together so as to form a leak-proof cell, characterized in that the feed plate (3) has two or more inlet ports (1, 2) for feeding the said liquid; a dam (4) attached to the periphery of the bottom surface of the feed plate (3) and situated near connecting orifices (5) connected to the inlet ports (1, 2) for enlarging the radius of the radial flow from the circular groove (6); and two or more outlet ports with enlarged openings (10, 12) in the feed plate.

2. A cell as claimed in Claim 1 in which the means for filtration includes a micropore membrane (9); a screen (17) for supporting the membrane; and a circular shoulder (18) extending upwardly from the bottom surface of the cavity of the filtrate plate (20) for supporting the screen.

### Revendications

1. Une cellule à flux radial du type ayant une plaque d'alimentation (3) avec des dispositifs d'entrée et de sortie pour un liquide hétérogène nécessitant une filtration; une rainure circulaire (6) pour recevoir par des orifices de raccordement ledit liquide à partir du dispositif d'entrée et produire ainsi un flux radial dudit liquide; une plaque de filtrat (20) avec un dispositif de filtration (9); et un dispositif d'étanchéité (22) pour assujettir ensemble la plaque d'alimentation (3) et la plaque de filtrat (20) de façon à former une cellule étanche, caractérisée en ce que la plaque d'alimentation (3) a deux trous d'entrée (1, 2) ou plus pour l'alimentation dudit liquide; une digue (4) fixée au pourtour de la surface de fond de la plaque d'alimentation (3) et située près des orifices de reccordement (5) raccordés aux trous d'entrée (1, 2) pour élargir le rayon du flux radial à partir de la rainure circulaire (6); et deux trous d'entrée ou plus ayant des ouvertures élargies (10, 12) dans la plaque d'alimentation.

2. Une cellule comme revendiqué dans la revendication 1 dans laquelle le dispositif de filtration comprend une membrane microporeuse (9); une toile (17) pour supporter la membrane; et un épaulement circulaire (18) s'étendant vers le haut à partir de la surface de fond de la cavité de la plaque de filtrat (20) pour supporter la toile.

**Patentansprüche**

1. Zelle mit radialem Fluß des Typs, der eine Aufgabeplatte (3) mit Einlaß- und Auslaßrichtungen für eine heterogene, zu filtrierende Flüssigkeit, eine kreisförmige Nut (6) zur Aufnahme der Flüssigkeit von der Einlaßeinrichtung über Verbindungsöffnungen und zur Erzeugung eines radialen Flusses dieser Flüssigkeit, eine Filtergutplatte (20) mit Filtereinrichtungen (9) und eine Dichtungseinrichtung (22) zur festen Verbindung der Aufgabeplatte (3) und der Filtergutplatte (20) miteinander unter Bildung einer leckdichten Zelle hat, dadurch gekennzeichnet, daß die Aufgabeplatte (3) zwei oder mehr Einlaßöffnungen (1, 2) zum Zuführen der Flüssigkeit hat, daß ein Damm (4) am Umfang der Bodenfläche der Aufgabeplatte (3) angebracht ist und sich in der Nähe der Verbindungsöffnungen (5) befindet, die mit den Einlaßöffnungen (1, 2) zur Vergrößerung des Radius des radialen Flusses von der kreisförmigen Nut (6) verbunden ist, und daß zwei oder mehr Auslaßöffnungen mit vergrößerten Öffnungen (10, 12) in der Aufgabeplatte vorgesehen sind.

2. Zelle nach Anspruch 1, dadurch gekennzeichnet, daß die Filtriereinrichtung eine mikroporöse Membrane (9), einen Filter (17) zum Stützen der Membrane und eine kreisförmige Schulter (18) enthält, die sich von der Bodenfläche des Hohlraums der Filtergutplatte (20) zum Stützen des Filters nach oben erstreckt.

Fig.1.

Fig.2.

Fig.3.

Fig.4.

Fig.5.

Fig.6.

Fig.7